(19) Europäisches Patentamt
European Patent Office
Office européen des brevets

(11) Numéro de publication : **0 028 555**
**B1**

(12) **FASCICULE DE BREVET EUROPÉEN**

(45) Date de publication du fascicule du brevet : **22.06.83**

(51) Int. Cl.³ : **C 07 C 89/04**, C 07 C 91/06

(21) Numéro de dépôt : **80401477.7**

(22) Date de dépôt : **17.10.80**

(54) **Procédé de purification des N,N dialkylaminoéthanols par hydrogénation.**

(30) Priorité : **31.10.79 FR 7926989**

(43) Date de publication de la demande : **13.05.81 Bulletin 81/19**

(45) Mention de la délivrance du brevet : **22.06.83 Bulletin 83/25**

(84) Etats contractants désignés : **BE DE FR GB IT NL SE**

(56) Documents cités : **NEANT**

(73) Titulaire : **P C U K PRODUITS CHIMIQUES UGINE KUHLMANN**
**Service Propriété Industrielle Tour Manhattan**
**F-92087 Paris La Défense 2 Cédex 21 (FR)**

(72) Inventeur : **Ghenassia, Elie François Marius**
**100, rue Benoite Vincent**
**F-62400 Béthune (FR)**
Inventeur : **Lakodey, André Jean**
**37, rue de Lillers**
**F-62920 Chocques (FR)**

(74) Mandataire : **Foiret, Claude et al**
**P C U K Produits Chimiques Ugine Kuhlmann Service Propriété Industrielle Tour Manhattan - Cedex 21**
**F-92087 Paris la Défense 2 (FR)**

**EP 0 028 555 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé de purification des N,N,dialkylaminoéthanols par hydrogénation

La présente invention concerne un procédé de purification des alcanolamines répondant à la formule générale

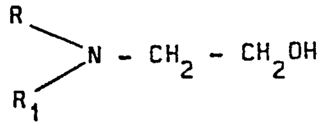

dans laquelle R et $R_1$ sont des radicaux alkyls de structure identique ou non, linéaire ou ramifiée, de la forme méthyl, éthyl, propyl, isopropyl, butyl, isobutyl, tertiobutyl, etc...

Suivant le mode de préparation communément utilisé, l'oxyde d'éthylène réagit sous pression en phase liquide à 100-150 °C avec les dialkylamines R—NH—$R_1$ où R et $R_1$ sont des radicaux alkyls identiques ou non, linéaires de la forme méthyl, éthyl, propyl, etc... ou ramifiés de la forme isopropyl, isobutyl, tertiobutyl, etc... La réaction conduit aux composés d'addition à structure

où n = 1, 2, 3 et plus.

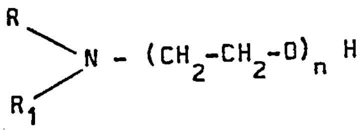

Parmi les produits obtenus, les plus intéressants, par leurs utilisations, sont les N,N,dialkylamino-éthanols

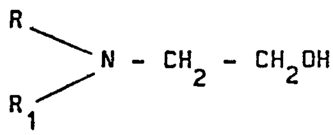

facilement séparables de leurs homologues supérieurs par distillation. Un certain nombre de ces composés monoéthoxylés sont transformés en esters d'acides carboxyliques insaturés utilisés dans la préparation d'agents cationiques de floculation pour le traitement des eaux usées. C'est en particulier le cas des esters des acides acryliques et méthacryliques, employés comme monomères pour donner des homopolymères ou bien avec d'autres composés acryliques ou méthacryliques pour conduire à des copolymères.

Au cours de ces homo ou copolymérisations, la présence d'impuretés entraîne une réaction parasite conduisant à une gélification du milieu, très gênante dans l'application finale de ces produits dans le traitement des eaux. L'apparition du gel insoluble peut être reliée à la présence d'impuretés contenues initialement dans les alcanolamines entrant dans la préparation de ces esters d'acide insaturés utilisés pour préparer les polymères cationiques. Pour tenter de supprimer cet inconvénient, les techniques classiques de purification, en particulier la distillation, se sont révélées insuffisantes. Pratiquées soit sur l'aminoalcool, soit sur son ester insaturé monomère, ces techniques ne permettent pas d'amener la teneur des impuretés à l'origine de la réticulation parasite, à une valeur compatible avec les applications, soit parce que trop difficiles à réaliser techniquement, soit parce que peu réalisables économiquement étant donné la consommation d'énergie entraînée ou l'investissement nécessaire.

Le procédé selon l'invention consiste à faire subir aux N,N,dialkylaminoéthanols, avant une purification par distillation, un traitement d'hydrogénation catalytique en phase hétérogène, pour une purification préalable. Cette purification préalable permet d'abaisser la teneur en impuretés à une valeur suffisamment faible pour qu'une distillation peu onéreuse en énergie, effectuée par exemple dans une colonne à rectifier de 8 à 12 plateaux réels avec un taux de reflux de 10 pour 1, permette d'obtenir un aminoalcool de pureté parfaitement adaptée à son application.

On peut réaliser la purification par hydrogénation catalytique de façon discontinue sous agitation ou non en phase liquide dans des réacteurs fonctionnant à pression atmosphérique ou sous pression, le mélange aminoalcool-catalyseur étant parcouru par un courant d'hydrogène.

Cette purification peut également se faire en continu dans des réacteurs à co-courant descendant sur les catalyseurs généralement en grains ou extrudés avec introduction d'hydrogène et des alcanolamines, ces dernières pouvant être partiellement ou totalement à l'état gazeux selon les températures et débits des N,N,dialkylaminoéthanols et d'hydrogène. On peut procéder selon la technique connue dans laquelle les deux réactifs pénètrent par le haut du réacteur constitué par un tube cylindrique et s'écoulent de haut en bas. On peut aussi réaliser l'hydrogénation en phase liquide, dans un réacteur constitué par un tube

cylindrique contenant l'aminoalcool et le catalyseur généralement en grains ou extrudés et dans lequel le contact intime, de l'hydrogène et du N,N,dialkylaminoéthanol à purifier, tous deux introduits à la base du réacteur, est assuré par la mise en œuvre d'un courant d'hydrogène en excès. La technique classique du contre-courant peut aussi être utilisée en introduisant l'aminoalcool par le haut du réacteur et le courant d'hydrogène par le bas.

Les catalyseurs utilisés pour les hydrogénations en général conviennent dans le cas de la présente invention. On utilise de préférence les métaux du groupe VIII de la table périodique et plus particulièrement le nickel réduit, le nickel de Raney, le cobalt de Raney ou encore les métaux nobles comme le platine, le rhodium, le palladium. Suivant le mode d'hydrogénation choisi, le catalyseur peut être utilisé sous forme de grains ou sous forme de particules finement divisées. Sous forme de grains, le catalyseur, déposé sur un support comme l'alumine, le charbon actif, le kieselguhr, la pierre ponce, ou toute autre substance support, se présente habituellement en grains de 1 à 3 mm ou d'extrudés de 3 à 5 mm. On utilise habituellement le catalyseur sous cette forme dans le cas de l'hydrogénation dans une colonne à co-courant descendant ou à contre-courant, ou encore en phase liquide. Sous forme de particules finement divisées, le catalyseur est généralement déposé sur des substances supports identiques à celles employées dans les catalyseurs en grains précédents. On utilise habituellement les catalyseurs sous cette forme dans le cas de l'hydrogénation en discontinu.

L'hydrogénation peut être réalisée en présence d'hydrogène en excès, sous pression atmosphérique ou éventuellement sous pression, cette dernière n'étant pas indispensable pour le bon fonctionnement de l'invention. La quantité d'hydrogène mise en œuvre dans un procédé opérant en continu peut être telle que le volume d'hydrogène, mesuré dans les conditions normales de température et de pression, traversant le bain ou le lit catalytique, soit de 0,1 à 300 fois le volume d'aminoalcool liquide à purifier, de préférence 0,5 à 200 fois.

Le débit de N,N,dialkylaminoéthanol ramené au volume de l'ensemble catalytique est de 0,1 à 5, de préférence 0,5 à 1, dans les réacteurs en continu pour une teneur en métal actif de 15 à 60 % en poids de l'ensemble catalytique métal plus support. Dans les réacteurs discontinus, la concentration en métal actif peut atteindre 0,005 à 1 % du poids de N,N,dialkylaminoéthanol à purifier.

La température d'hydrogénation est choisie en fonction de l'aminoalcool à purifier. Elle peut être comprise entre 50 et 175 °C, de préférence on fait en sorte qu'elle soit inférieure d'environ 10 à 50 °C au point d'ébullition du N,N,dialkylaminoéthanol à purifier, sous la pression choisie pour effectuer l'hydrogénation.

La pression d'hydrogénation recommandée est la pression atmosphérique qui permet d'utiliser un équipement industriel simple, bien que l'emploi de pression supérieure à la pression atmosphérique, par exemple de 1 à 10 bars intrinséquement favorable à l'hydrogénation, puisse être employée si l'on dispose de l'appareillage adapté.

L'hydrogénation peut se faire directement sur le distillat de la colonne de rectification des N,N, monoalcanolamines. Cependant il est préférable d'appliquer le traitement de purification sur le mélange brut issu du réacteur de synthèse car une distillation est de toute façon indispensable, particulièrement pour les N,N,diméthyl et le diéthylaminomonoéthanol, en vue de les séparer de leurs homologues supérieurs qui se forment au cours de la préparation des dialkylaminoéthanols. En effectuant l'hydrogénation avant la rectification, on élimine simultanément au cours de celle-ci, les impuretés provenant directement de l'alcoxylation et celles formées au cours de l'hydrogénation. L'hydrogénation peut aussi se faire sur les monoalcanolamines rectifiées, les produits qui se forment pendant l'hydrogénation n'influent pas sur la qualité des esters et polymères obtenus dans les étapes suivantes de l'application.

La présente invention est illustrée par les exemples suivants.

### Exemple 1

Dans un réacteur de 250 $cm^3$ en verre à double enveloppe dans laquelle circule un fluide caloporteur thermostaté, 150 g de diméthylaminoéthanol brut, provenant du réacteur de synthèse, sont hydrogénés par un courant d'hydrogène de 5 l/h N.T.P. en présence de 1,5 g de nickel Raney. Après 2 heures d'agitation à 100 °C, le mélange est filtré pour éliminer le catalyseur, distillé dans une colonne à garnissage de 10 plateaux réels, avec un taux de reflux de 10 pour 1. Le N,N,diméthylaminoéthanol distillé à 134 °C sous pression atmosphérique, est ensuite estérifié selon les méthodes classiques de transestérification par l'acrylate d'éthyle pour donner l'acrylate de diméthylaminoéthyle.

50 g de l'ester sont dissous dans 300 g de toluène et 0,40 g de peroxyde de benzoyle est ajouté sous atmosphère d'azote. La température est maintenue à 80 °C pendant 6 heures. Le polymère obtenu est soluble dans le toluène.

### Exemple 2

A titre comparatif, on répète l'exemple 1 à l'exception près qu'est utilisé un N,N,diméthylaminoéthanol n'ayant pas subi le traitement d'hydrogénation. On obtient finalement un polymère non soluble dans le toluène.

Exemple 3

Dans l'appareillage de l'exemple 1, 1,5 g de masse catalytique, à 0,5 % en poids de métal actif, constituée de palladium déposé sur charbon, est ajouté à 100 g de diméthylaminoéthanol brut. Un courant d'hydrogène de 5 l/h N.T.P. de débit traverse le réacteur, le catalyseur étant mis en suspension par agitation. Après 7 heures à 100 °C, le N,N,diméthylaminoéthanol hydrogéné est distillé, après élimination du catalyseur, dans une colonne à garnissage de 10 plateaux réels avec un taux de reflux de 10/1. Le distillat, après estérification par l'acrylate d'éthyle, est soumis au test de polymérisation en présence de peroxyde de benzoyle. Le polymère obtenu est soluble dans le toluène.

Exemple 4

Dans l'appareillage décrit dans l'exemple 1, 1,5 g de masse catalytique, à 0,5 % en poids de métal actif, constituée de platine déposé sur de l'alumine en poudre, est ajouté à 150 g de diéthylaminoéthanol. Après 7 heures à 120 °C, le produit hydrogéné est distillé, après élimination du catalyseur, sur une colonne à garnissage de 10 plateaux réels et le distillat bouillant à 84° sous une pression réduite de 50 mm de mercure, est estérifié par l'acrylate d'éthyle en acrylate de diéthylaminoéthyle. 50 g d'ester sont polymérisés en présence de 0,5 g de peroxyde de benzoyle dans 200 g de toluène. Après 6 heures à 80 °C sous atmosphère d'azote, le polymère obtenu est soluble dans le toluène.

En opérant dans les mêmes conditions, avec un acrylate de diéthylaminoéthyle préparé à partir d'un diéthylaminoéthanol distillé sur la colonne définie ci-dessus, mais non hydrogéné, la polymérisation conduit à un polymère non soluble dans le toluène.

Exemple 5

Dans une colonne à double enveloppe de 1,6 cm de diamètre intérieur et de 90 cm de hauteur, contenant 120 $cm^3$ de catalyseur constitué par des grains de 1 à 3 mm de nickel réduit déposé sur kieselghur, à 50 % en poids de nickel, on introduit 50 $cm^3$ par heure de N,N,diméthylaminoéthanol brut provenant du réacteur de synthèse, à co-courant descendant avec 5 litres par heure N.T.P. d'hydrogène. Le mélange des réactifs est préchauffé à 115-118 °C sur un lit de billes de verre de 30 cm de haut et la hauteur du lit catalytique atteint 56 cm. On opère à pression atmosphérique à la température de 115-118 °C. L'aminoalcool hydrogéné est recueilli au bas de la colonne et l'hydrogène non consommé peut ou non être recyclé. Le produit hydrogéné est distillé dans une colonne Oldershow de 12 plateaux avec un reflux de 10/1 et le distillat est soumis après estérification par l'acrylate d'éthyle, au test de polymérisation par le peroxyde de benzoyle selon l'exemple 1. Le polymère obtenu est soluble dans le toluène.

Exemple 6

Dans une colonne à double enveloppe de 1,6 cm de diamètre intérieur et de 90 cm de hauteur, contenant 120 $cm^3$ de catalyseur constitué par des grains de 1 à 3 mm de nickel déposé sur kieselghur à 50 % en poids de nickel, on introduit 200 $cm^3$ par heure de N,N,diméthylaminoéthanol brut provenant du réacteur de synthèse, à co-courant descendant avec 100 litres par heure N.T.P. d'hydrogène. Le mélange des réactifs est préchauffé à 115-118 °C sur un lit de billes de verre de 30 cm de haut et la hauteur du lit catalytique atteint 56 cm. On opère à pression atmosphérique à la température de 115-118 °C. L'aminoalcool hydrogéné est recueilli au bas de la colonne et l'hydrogène non consommé peut ou non être recyclé. Le produit hydrogéné est distillé dans une colonne Oldershow de 12 plateaux avec un reflux de 10/1 et le distillat est soumis après estérification par l'acrylate d'éthyle, au test de polymérisation par le peroxyde de benzoyle selon l'exemple 1. Le polymère obtenu est soluble dans le toluène.

Exemple 7

Dans une colonne à double enveloppe de 38 mm de diamètre intérieur et de 135 mm de haut, contenant 100 $cm^3$ de catalyseur constitué par des grains de 1 à 3 mm de nickel réduit déposé sur kieselghur à 50 % en poids de nickel, on introduit un débit de 50 $cm^3$/h de N,N,diméthylaminoéthanol liquide brut provenant du réacteur de synthèse. La hauteur du lit catalytique atteint 9 cm et une couche de billes de verre de 4 cm limite le volume mort supérieur du réacteur d'hydrogénation. L'aminoalcool brut et l'hydrogène à raison de 10 l/h N.T.P. pénètrent pas le bas du réacteur. L'hydrogénation a lieu à 115-120 °C, à pression atmosphérique. Le mélange N,N,diméthylaminoéthanol-hydrogène sortant par le haut du réacteur, est reçu dans un séparateur d'où l'hydrogène non consommé peut être ou non recyclé au bas du réacteur d'hydrogénation. Le N,N,diméthylaminoéthanol hydrogéné, mais contenant encore des termes supérieurs tels que le N,N,diméthylaminodiéthanol, est rectifié dans une colonne Oldershow de 12 plateaux théoriques avec un reflux de 10/1. Le distillat bouillant à 134 °C, constitué de N,N,diméthylaminoéthanol est estérifié en acrylate de diméthylaminoéthyle puis polymérisé en présence de peroxyde de benzoyle selon l'exemple 1. Le polymère obtenu est soluble dans le toluène.

L'ester acrylique préparé à partir du N,N,diméthylaminoéthanol brut provenant du réacteur de synthèse et simplement distillé dans la colonne Oldershow de 12 plateaux réels, avec un taux de reflux de 10/1, conduit, après polymérisation, à un polymère non soluble dans le toluène.

Exemple 8

Dans la colonne à double enveloppe de 1,6 cm de diamètre intérieur et de 90 cm de hauteur, on introduit 100 $cm^3$ de catalyseur constitué par des extrudés de nickel déposé sur kieselghur, de 2 à 5 mm de longueur et de 2 à 3 mm de diamètre. Le lit catalytique de 50 cm de hauteur est surmonté d'une couche de billes de verre de 25 cm utilisés comme préchauffeur. Dans la double enveloppe circule un fluide caloporteur permettant de régler la température d'hydrogénation.

Par le haut du réacteur sont introduits, par heure, à co-courant descendant, 50 $cm^3$ de N,N, diméthylaminoéthanol préalablement distillé dans une colonne à garnissage de 10 plateaux réels avec un reflux de 10 à 1, et 5 l/h N.T.P. d'hydrogène. La température du lit catalytique est maintenue à 115 °C sous pression atmosphérique. A la sortie du tube d'hydrogénation, le mélange d'aminoalcool purifié et d'hydrogène passe dans un séparateur d'où l'hydrogène non transformé peut ou non être recyclé à l'entrée de l'hydrogénateur. Le N,N,diméthylaminoéthanol est ensuite estérifié tel quel par l'acrylate d'éthyle et polymérisé selon les conditions de l'exemple 1. Le polymère obtenu est soluble dans le toluène.

Le test de polymérisation est effectué sur l'ester du N,N,diméthylaminoéthanol distillé et non hydrogéné. Le polymère obtenu est insoluble dans le toluène.

**Revendications**

1. Procédé de purification des N,N,dialkylaminoéthanols caractérisé en ce qu'avant une purification par distillation, on leur fait subir un traitement d'hydrogénation catalytique en phase hétérogène, pour une purification préalable.

2. Procédé selon la revendication 1 caractérisé en ce que le catalyseur est choisi parmi les métaux du groupe VIII de la table périodique.

3. Procédé selon la revendication 2 caractérisé en ce que le catalyseur est choisi parmi le nickel réduit, le nickel de Raney, le cobalt de Raney, le platine, le rhodium, le palladium.

4. Procédé selon l'une des revendications 1 à 3 caractérisé en ce que dans un réacteur en continu le débit de N,N,dialkylaminoéthanol, ramené au volume de l'ensemble catalytique, est de 0,1 à 5 pour une teneur en métal actif de 15 à 60 % en poids de l'ensemble catalytique.

5. Procédé selon l'une des revendications 1 à 3 caractérisé en ce que la teneur en métal actif dans un réacteur en discontinu représente de 0,005 à 1 % du poids de N,N,dialkylaminoéthanol à purifier.

6. Procédé selon l'une des revendications 1 à 5 caractérisé en ce que le volume d'hydrogène, mesuré dans les conditions normales de température et de pression, traversant le milieu réactionnel est de 0,1 à 300 fois le volume de N,N,dialkylaminoéthanol liquide à purifier.

7. Procédé selon l'une des revendications 1 à 6 caractérisé en ce que l'hydrogénation s'effectue sous une pression comprise entre la pression atmosphérique et 10 bars.

8. Procédé selon l'une des revendications 1 à 7 caractérisé en ce que la température d'hydrogénation est comprise entre 50 et 175 °C.

9. Procédé selon la revendication 8 caractérisé en ce que la température d'hydrogénation est inférieure de 10 à 50 °C au point d'ébullition sous la pression d'hydrogénation du N,N,dialkylaminoéthanol à purifier.

**Claims**

1. Process for the purification of N,N-dialkylaminoethanols, characterised in that, before purification by distillation, they are subjected to a catalytic hydrogenation treatment in the heterogeneous phase by way of preliminary purification.

2. Process according to Claim 1, characterised in that the catalyst is chosen from amongst the metals of group VIII of the periodic table.

3. Process according to Claim 2, characterised in that the catalyst is chosen from amongst reduced nickel, Raney nickel, Raney cobalt, platinum, rhodium and palladium.

4. Process according to one of Claims 1 to 3, characterised in that, in a continuously operating reactor, the flow of N,N-dialkylaminoethanol, based on the volume of the whole of the catalyst, is 0.1 to 5 for a content of active metal of 15 to 60 % by weight of the whole of the catalyst.

5. Process according to one of Claims 1 to 3, characterised in that the content of active metal in a discontinuously operating reactor represents from 0.005 to 1 % of the weight of N,N-dialkylaminoethanol to be purified.

6. Process according to one of Claims 1 to 5, characterised in that the volume of hydrogen, measured

under normal temperature and pressure conditions, passing through the reaction medium is 0.1 to 300 times the volume of liquid N,N-dialkylaminoethanol to be purified.

7. Process according to one of Claims 1 to 6, characterised in that the hydrogenation is carried out under a pressure between atmospheric pressure and 10 bars.

8. Process according to one of Claims 1 to 7, characterised in that the hydrogenation temperature is between 50 and 175 °C.

9. Process according to Claim 8, characterised in that the hydrogenation temperature is 10 to 50 °C below the boiling point, under the hydrogenation pressure, of the N,N-dialkylaminoethanol to be purified.

**Ansprüche**

1. Verfahren zur Reinigung von N,N-Dialkylaminoäthanolen, dadurch gekennzeichnet, daß man sie vor der Reinigung durch Destillation zur Vorreinigung einer katalytischen Hydrierungsbehandlung in heterogener Phase unterwirft.

2. Verfahren nach Anspruch 1, dadurch gekennzeichnet, daß der Katalysator unter den Metallen der VIII. Gruppe des Periodischen Systems gewählt ist.

3. Verfahren nach Anspruch 2, dadurch gekennzeichnet, daß der Katalysator unter reduziertem Nickel, Raney-Nickel, Raney-Kobalt, Platin, Rhodium oder Palladium gewählt ist.

4. Verfahren nach einem der Ansprüche 1 bis 3, dadurch gekennzeichnet, daß der Durchsatz von N, N-Dialkylaminoäthanol, bezogen auf das Volumen der Gesamtheit des Katalysators, in einem Kontinuierlichen Reaktor 0,1 bis 5 bei einem Gehalt des aktiven Metalls von 15 bis 60 Gew.% der Gesamtheit des Katalysators beträgt.

5. Verfahren nach einem der Ansprüche 1 bis 3, dadurch gekennzeichnet, daß der Gehalt an aktivem Metall in einen diskontinuierlichen Reaktor 0,005 bis 1 Gew.% des zu reinigenden N,N-Dialkalaminoäthanols beträgt.

6. Verfahren nach einem der Ansprüche 1 bis 5, dadurch gekennzeichnet, daß das unter normalen Druck- und Temperaturbedingungen gemessene Wasserstoff-Volumen, welches das Reaktionsgemisch durchströmt, das 0,1 bis 300-fache des Volumens des flüssigen, zu reinigenden N,N-Dialkylaminoäthanols beträgt.

7. Verfahren nach einem der Ansprüche 1 bis 6, dadurch gekennzeichnet, daß die Hydrierung bei einem Druck zwischen Atmosphärendruck und 10 bar durchgeführt wird.

8. Verfahren nach einem der Ansprüche 1 bis 7, dadurch gekennzeichnet, daß die Hydrierungstemperatur zwischen 50 und 175 °C liegt.

9. Verfahren nach Anspruch 8, dadurch gekennzeichnet, daß die Hydrierungstemperatur 10 bis 50 °C unter dem Siedepunkt des zu reinigenden N,N-Dialkylaminoäthanols beim Druck der Hydrierung liegt.